# EUROPEAN PATENT SPECIFICATION

(11) **EP 0 960 599 B1**
(45) Date of publication and mention of the grant of the patent: **14.11.2001**
(21) Application number: 99109035.8
(22) Date of filing: 07.05.1999
(51) Int. Cl.: A61B 17/02

(54) **Myocardial stabilizer**
Stabilisator für die Herzchirurgie
Stabilisateur du myocarde

(30) Priority: 28.05.1998 CZ 164098
(43) Date of publication of application: 01.12.1999
(73) Proprietor: MUDr MILAN KRAJICEK CSc, 149 00 Praha 4 (CZ)
(72) Inventor: MUDr MILAN KRAJICEK CSc, 149 00 Praha 4 (CZ)
(74) Representative: Ksoll, Peter, Dr.-Ing.

(56) References cited:
- EP-A- 0 791 330

## Description

### Scope of technique

The myocardial stabilizer according to the invention solves the way of the immobilization of a definite part of a working heart so as to enable an indicated performance on heart wall structures, namely coronary arteries.

### Technique state-of-the-art

For illustration may be used the most frequent cardiosurgical performance which means indisputably a direct revascularization of myocard through operation on coronary arteries Most of them are still performed in extra-corporeal circulation on a not beating heart. Over recent years however, in indicated cases a method has more and more often been used, where reconstruction, which means peripheral anastomosis of vascular substitute is feasible on working heart without stopping the heart and using extra-corporeal circulation. This means, of course, an additional risk.

Anastomosis of the vascular substitute, which is mostly one of the patient's veins to in general delicate coronary artery , is a delicate performance. The substantial and necessary condition for the successof an operation is a perfect technical execution. The movement of the coronary artery, located under the epicardium, depends of the heart muscle own movement. As the frequency of heart beats can't be decreased under a certain level, this method has its physiological limits. Thus there is a certain contradiction in the necessity of a perfect placing of stiches into edges of a longitudinally opened coronary artery on one hand and a continuous movement of the operation field on the other hand.

These days there are two devices applied to immobilize the coronary artery's part to be operated on during the performance. In principle it is something like a fork with arms either joined together, or individually controlled. One known method to calm down the movement of the heart is to presson a specified point of the cardial muscle, the other consists in devices with contacting surfaces provided with absorbing holes whereby a respective area gets sucked into the device within the contact point of the device and cardial muscle.

These devices are much used today. However they have their disadvantages. Handling a device using pressure cannot exclude pressure damage of the cardiac wall structures, especially when the operation takes a longer time and a high pressure is applied. Another disadvantage of this method isthe limited possibility to adjust the pressure, the tendency of the cardiac muscle to move and and to slip out under the applied devices. These devices are in practice not usable and impossible use for all new reconstructed coronary arteries with regard to their anatomical position.

Devices using the vacuum principle are an improvement with regard to the above mentiond devices . The stabilizer OCTOPUS is considered to be the most acceptable solution known in the state of the art. This stabilizer is characeterized by two independent flexible arms with end sections of various forms provided with various number of absorption holes. The arms bases and the whole suction mechanism is placed outside the operating table, whereby a surgeon handles only the end sections, which he can place as neededand then clamp the wall on given area through connecting of suction. This enables an access even to coronary arteries on the opposite side of the heart. Disadvantage of this way is again a possible damage of the cardiac muscle structures through a negative pressure and generating small subepicardial and myocardial bleeding, whereby late sequelae are not yet quite clear. In principle there arises a range of small phenomea called in colloquial language (and in another locality) as "suckflecks".

Both devices are well known in various modifications and are also comparatively expensive.

EP 0 791 330 A2 shows a variety of surgical instruments and methods for stabilizing a beating heart during coronary artery bypass graft surgery. Typical for these instruments is that the surgeon contacts the heart with the stabilizing means, assesses the degree of movement of the anastomosis site and exerts a force on the stabilizing means such that the contraction of the beating heart causes minimal excess motion at the surgery site. The stabilizing means may be attached to a rigid support or may be attached to a semi-rigid support which is rendered motionless mechanically, chemically, or by human intervention.

### Principle of the invention

The device according to the invention uses the effect of magnetic induction in a stationary magnetic field. This is to say that the device has two individual parts. The first part is a fibre of ferromagnetic material or magnetically hard material, wherein the fibre is drawn on a desired point and in a length through myocard. The second part is an arm, or fork with either a permanent magnet or an electromagnet.

Both, the principle of the invention and its practical use are relatively simple. Let's have a model stationary magnetic field, realized approximately by the field between discordant extensive poles of the magnet. A magnetic field is characterized in an vectorial variable called magnetic induction. Unit of the magnetic induction is Tesla T and the size of the magnetic induction of the field, e.g. near current permanent magnet is 10⁻¹ to 10⁻² T. Of course there are magnets, in principle electromagnets to be made where field size values reach Tesla units order. As example can be given that the Earths magnetic field has within our geographic latitudes a magnetic induction of about 10⁻⁵ T .

For the device according to the invention it is not important how the magnetic field is generated, whether it is generated by permanent-, or by electromagnetic induction, whether through a classic electromagnet or selenoid. Both, theory and practice in using a magnetic field are a widespread independent branch, whereas the principle of its effect is over the same.

The size of the magnetic induction depends to considerable extent on s.c. environmental permeability within the magnetic field. This can be demonstrated on the example of an electromagnet coil. Steel has a considerably high relative permeability and that is why the magnetic induction of the coil wound on a closed steel core is much higher than by the same corefree coil. According to the value of the relative permeability depending on arrangement of magnetic fields of individual stoms three types of materials are considered. Of the highest value are s.c. ferromagnetic materials (matters), atomic arrangement of which considerably magnifies the magnetic field. For example the relative permeability of steel is µr = 8.000.

Even a weak external magnetic field of ferromagnetic materials is sufficient to generate such arrangements of atoms, that the magnetic field gets magnified. The material gets magnetized and the magnetic field remains therein though external effect becomes extinct. Despite the number of ferromagnetic materials is not high (iron, cobalt, nickel, or their alloys as well as those not containing these elements), they have a considerable practical importance. It is, however, to realize that ferromagnetism of the stuffs is shown only in their cristalline state. It is therefore property of the material structure and not of individual atoms. Among ferromagnetic materials are classified also those ferrimagnetic - ferrits. They are compounds of iron oxides together with oxides of other metals, such as molybden. Their relative permeability is µr = 10² to 10³. They are largely applied especially as permanent magnets.

Referring to above mentioned the principle of use can be understood. To say in general, on the point of required immobilization of the cardiac muscle are drawn several, not less than two fibres made of ferromagnetic material through this muscle. A contact figure capable to induce magnetic field either on the principle of the permanent magnet, or electromagnet is applied to them on the external side, such a figure may be either in form of individual arms or those magnetically connected or of variety shapes. In case that the magnetic field is intensive enough, the fibre made of the ferromagnetic material is drawn to the magnet arm making the part within arms span of the cardiac muscle immobilized. The arms of the basic magnet may in inflexible or flexible form be stretched either from the base out of the operating field, or fixed by action of the magnetic field directly in the operating field to a frame made of ferromagnetic material. The cardiac muscle does not get damaged by drawing through of the fibre as stimulation electrodes are anyway drawn through there prior to the termination of the performance and an appropriate control of the magnetic field intensity may also affect the power needed for the fibre to be drawn to the magnet minimizing thus any casual danger of the pressure damage.

### Review of figures

- Fig. 1: Schematically shows two magnet arms getting out of a electromagnet core.
- Fig. 2a: Fibres made of ferromagnetic material and location of their possible drawing through the cardiac muscle.
- Fig. 2b: Two types of ferromagnetic fibres and some of possible levels for drawing through the cardiac wall along an horizontal axis.
- Fig. 3: One magnet arm along with the fibre made of ferromagnetic material, where arrows show direction of the fibre drawn to the magnet.
- Fig. 4: Arms of independent electromagnets with possible connection of each other with a nonmagnetic link.
- Fig. 5a, 5b, 5c: Some possible forms of an immobilized area between magnet arms.
- Fig. 6: Use of a permanent magnet.
- Fig. 7: View of an operating field with arms fixed to a frame made of ferromagnetic material.

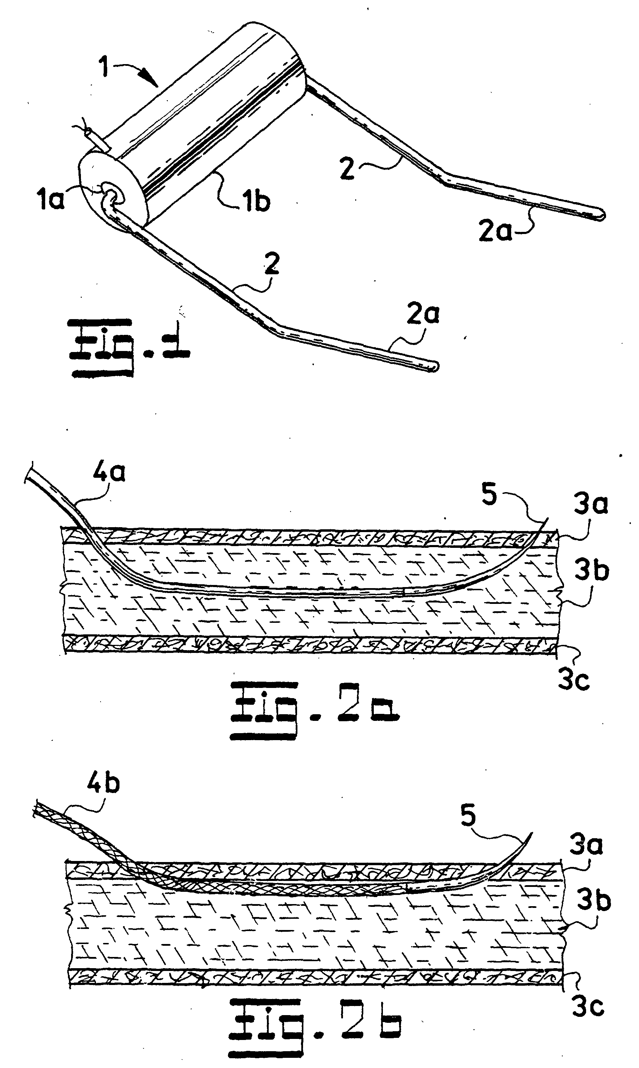

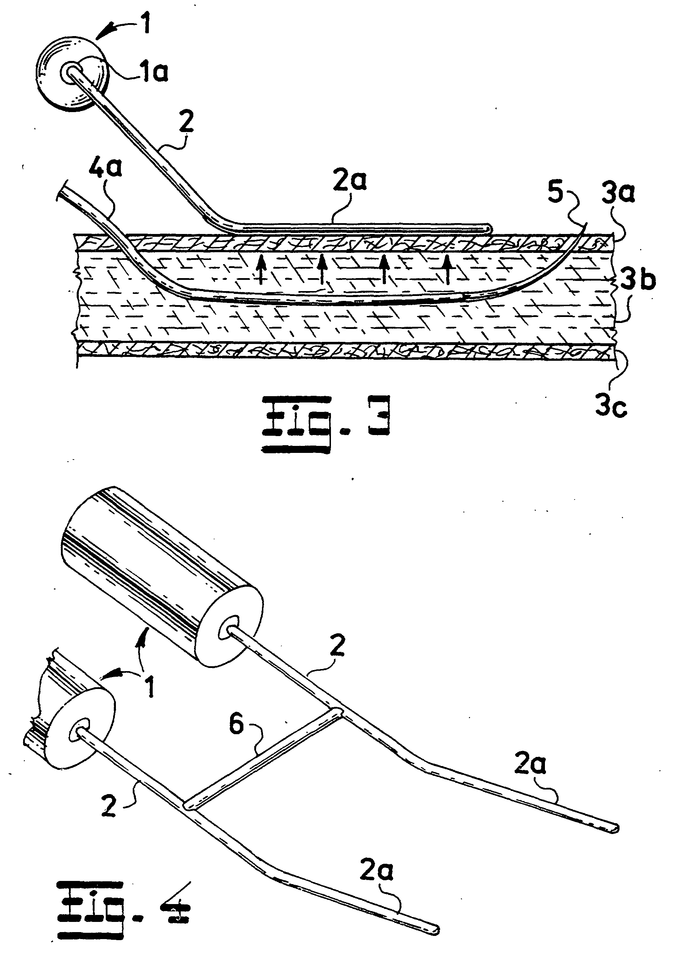

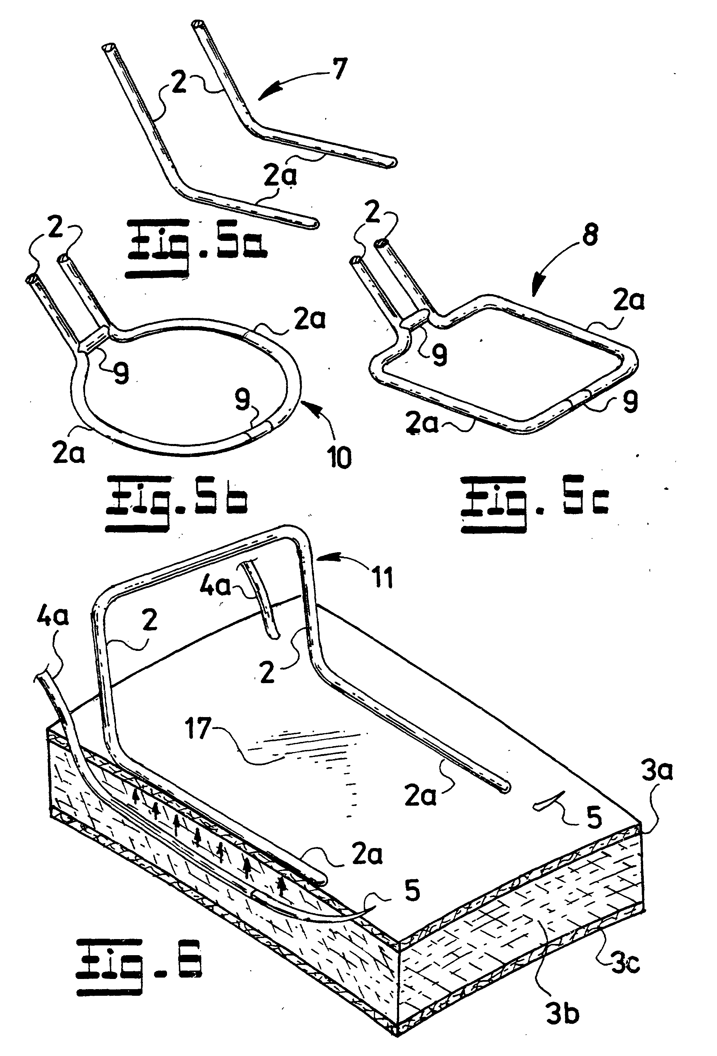

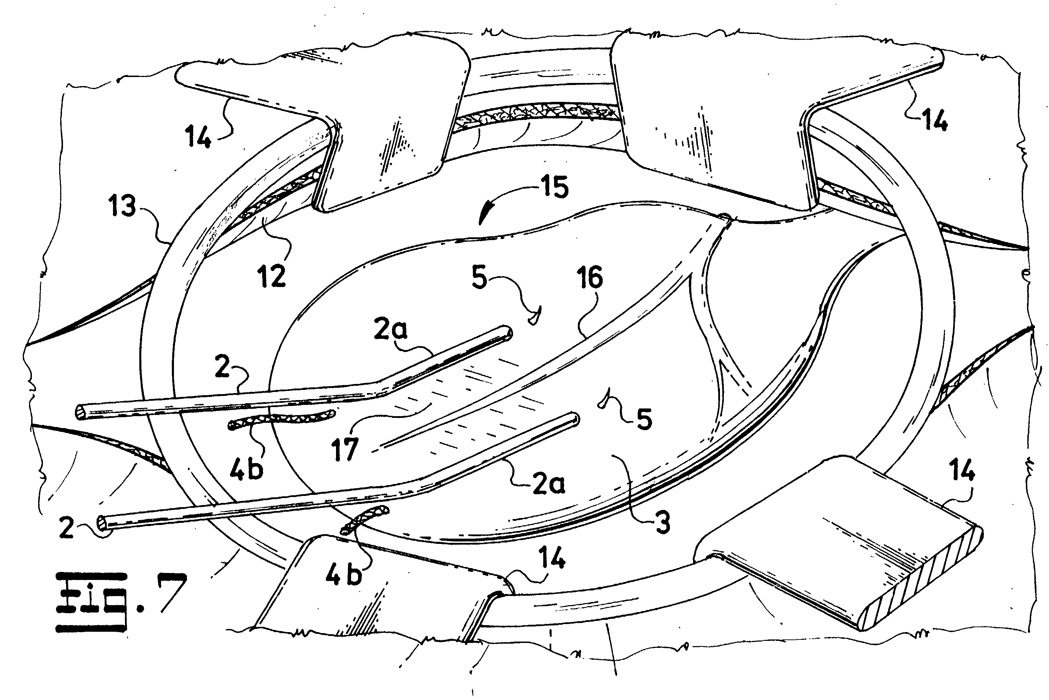

### Explanation of relating marks

- 1 -: electromagnet
- 1a -: core of electromagnet
- 1b -: electromagnet coil
- 2 -: arm coming from electromagnet core
- 2a -: terminal contact surface
- 3 -: myocard cardial muscle
- 3a -: epicard - top surface of the cardiac muscle
- 3b -: myocard - muscle layer itself
- 3c -: endocard - internal cardial muscle structure
- 4a -: monofilamentous ferromagnetic fibre drawn through myocard close to epicardial structure
- 4b -: mutifilamentous ferromagnetic fibre drawn through myocard in the center of its thickness
- 5 -: fibre forcing up needle
- 6 -: non-magnetic link of two magnetic arms
- 7 -: two independent magnetic arms
- 8 -: shape contact surfaces of the magnetic arms connected with non-magnetic links
- 9 -: non-magnetic connector
- 10 -: some other possible shapes of contact surfaces of two magnetic arms connected with a non-magnetic connector
- 11 -: permanent magnet
- 12 -: open sternum
- 13 -: rigid fixation frame made of ferromagnetic material
- 14 -: rigid hooks supporting operating field
- 15 -: heart
- 16 -: a coronary artery
- 17 -: immobilized section of myocardial structures

### Examples of invention workmanship

1. From a core 1a of an electromagnet 1 are carried two arms 2 ended with parallel bent contact surfaces 2a. A fibre 4a made of ferromagnetic material is drawn through a myocard 3b at a distance from a second fibre 4a drawn through equal to the contact surfaces 2a of the arms 2 of the magnet 2. After the electromagnet 1, capable to generate magnetic induction of at least 10⁻²T, is turned on the fibres 4a are drawn towards the contact surfaces 2a, whereby an area of a cardiac muscle area between the contact surfaces 2a gets immobilized.
2. Independent unlinked arms 2 of a magnet are each equipped with own independent electromagnets 1 and are brought in bent arrangement with a base out of the operating table from countersides into operating field. Two fibres 4a, 4b are individually brought into an desired area of a heart 15 into the myocard 3b at a respective distance and length. To the fibres 4a, 4b are individually attached contact surfaces 2a of the individual arms 2 of the magnet 1 with a generated magnetic induction rating min. of 10⁻² T. Fibres 4a, 4b are drawn through to contact surfaces 2a of the magnets arms 2 and an area of the heart 15 limited by these contact surfaces 2a is fixed.
3. A permanent magnet 11 with bent arms 2 in form of a double-armed fork is applied to the heart 15 on the same point where fibres 4a were previously drawn through myocard 3b at the same distance as the arms 2 of this fork of the permanent magnet 11 capable to generate magnetic induction of at least 10⁻² T. The fibres 4a being applied to are immediately drawn towards the arms 2 of the permanent magnet 11 and an area 17 of the cardial muscle 3b between them is fixed.
4. Two independent permanent magnets 11 are fixed on the independent flexible arms, whose base is placed out of the operating table. On points of desired immobilization of the cardiac muscle 3 are fibres 4a, 4b introduced into myocard 3b at desired distance from each other and at desired length. An area 17 between the fibres 4a, 4b gets immobilized after independent permanent magnets 11 have been brought over those fibres 4a, 4b, whereby the fibres 4a, 4b are immediately drawn to the permanent magnets 11.
5. A rigid circular frame 13 made of the ferromagnetic material is attached to an operating field 12 and thereon are fixced by magnetic induction on one hand dilatory spoons 14 made of the same material, and on the other hand central parts of the arms with either electromagnets, or permanent magnets on a point enabling easy access to an heart area 15 set fixed in the same way as in examples 1 to 4.

## Claims

1. Device for local stabilization a cardiac muscle **characterized in that** it consists of two physically independent but functionally dependent parts, whereby a first part comprises a magnet source (1, 11) provided with bent arms (2) whose end sections form contact surfaces (2a) that are positioned onto the external surface of a heart (15) at a length desired for immobilization, and a second part that consist of fibres (4a, 4b) made of ferromagnetic material, whereby each fibre (4a, 4b) is brought into the cardiac muscle (3) by means of a needle (5) at a length corresponding to the contact surfaces (2a) of the arms (2), so that putting the contact surfaces (2a) of the arms (2) over the introduced ferromagnetic fibres (4a, 4b) will cause drawing each fibre (4a, 4b) towards the contact surfaces (2, 2a), whereby immobilization of a heart area (15) is achieved by introducing of at least two ferromagnetic fibres (4a, 4b) into the edges of the heart area (15) to be immobilized, while each ferromagnetic fibre (4a, 4b) is independently drawn to one arm (2).

2. Device according to claim 1 **characterized in that** the magnet source is a permanent magnet (11) which comprises two arms (2) that are formed like a fork, whereby the shape and the mutual distance of these arms (2) as well as the length of their contact surfaces (2a) determines width and length of an area (17) of the heart (15) to be immobilized and wherein each ferromagnetic fibre (4a, 4b) is placed in a mutual distance to the arms (2), while both fibres are equally drawn towards the arms by the applied magnetic field of the permanent magnet (11), which results in immobilization of the area (17).

3. Device according to claim 1 and 2 **characterized in that** the contact surfaces (2a) of the arms (2) are designed as a rectangle (8) provided with non-magnetic connection pieces (9).

4. Device according to claim 1 and 2 **characterized in that** the contact surfaces (2a) of the arms (2) are designed in a circular form (10) with non-magnetic connection pieces (9).

5. Device according to the claims 1, 2, 3 and 4, **characterized in that** the magnet source is an electromagnet (1) with a core (1a).

6. Device according to claim 5 **characterized in that** two parallel bent arms (2) with contact surfaces (2a) inducing a magnetic field to a surface of a heart (15) come out from ends of the core (1a) of electromagnet (1).

7. Device according to claims 1, 2, 3, 4, 5 and 6 **characterized in that** the arms (2) of the magnetic field inducing part are flexible, whereby a magnetic field applying base is placed out of a operation field (12), wherein it is possible to adjust the arms (2) in regard of their mutual position against the operation field (12).

8. Device according to claims 1, 2, 3, 4, 5, 6 and 7 **characterized in that** it consists of a rigid circular frame (13) made of ferromagnetic material, equipped with removable dilatable spoons (14) made of a magnetized ferromagnetic material, so that the spoons (14) and parts of magnetic field inducing components are drawn by an induced magnetic field on any point to the ferromagnetic circular frame (13).

9. Device according to claims 1, 2, 3, 4, 5, 6 and 7 **characterized in that** the part (4a, 4b) brought into the cardial muscle (3) can be a ferromagnetic double fibre in form of a safety-pin or ferromagnetic fixation tacks.

## Patentansprüche

1. Vorrichtung zur lokalen Stabilisierung eines Herzmuskels, **dadurch gekennzeichnet, dass** sie aus zwei physikalisch unabhängigen, in funktioneller Hinsicht jedoch abhängigen Teilen besteht, wobei ein erster Teil eine Magnetquelle (1, 11) mit gebogenen Armen (2) aufweist, deren Endabschnitte Kontaktflächen (2a) bilden, die auf der Außenfläche eines Herzens (15) über eine für die Immobilisierung erforderliche Strecke angebracht sind und ein zweiter Teil aus Fasern (4a, 4b), die aus ferromagnetischem Material hergestellt sind, besteht, wobei jede Faser (4a, 4b) mittels einer Nadel (5) über eine den Kontaktflächen (2a) der Arme (2) entsprechende Strecke in den Herzmuskel (3) eingebracht wird, so dass, wenn die Kontaktflächen (2a) der Arme (2) über den eingeführten ferromagnetischen Fasem (4a, 4b) platziert werden, jede Faser (4a, 4b) in Richtung auf die Kontaktflächen (2, 2a) gezogen wird, wodurch eine Immobilisierung eines Herzbereiches (15) erzielt wird, indem wenigstens zwei ferromagnetische Fasern (4a, 4b) in die Randbereiche des stillzulegenden Herzbereiches (15) eingeführt werden, wobei jede ferromagnetische Faser (4a, 4b) unabhängig zu einem Arm (2) gezogen wird.

2. Vorrichtung nach Anspruch 1, **dadurch gekennzeichnet, dass** die Magnetquelle aus einem Dauermagneten (11) besteht, der zwei wie eine Gabel geformte Arme (2) aufweist, wobei sowohl die Form und der gegenseitige Abstand dieser Arme als auch die Länge ihrer Kontaktflächen (2a) die Breite und Länge des Bereiches (17) des Herzens (15) bestimmt, der stillgelegt wird und wobei jede ferromagnetische Faser (4a, 4b) in einem bestimmten Abstand zu den Armen (2) angeordnet wird, wobei beide Fasern gleichzeitig durch das angelegte Magnetfeld des Dauermagneten (11) in Richtung auf die Arme gezogen werden, wodurch der Bereich (17) stillgelegt wird.

3. Vorrichtung nach den Ansprüchen 1 und 2, **dadurch gekennzeichnet, dass** die Kontaktflächen (2a) der Arme (2) als Rechteck (8) ausgebildet und mit unmagnetischen Verbindungsstücken (9) versehen sind.

4. Vorrichtung nach den Ansprüchen 1 und 2, **dadurch gekennzeichnet, dass** die Kontaktflächen (2a) der Arme (2) kreisförmig (10) ausgebildet und mit unmagnetischen Verbindungsstücken (9) versehen sind.

5. Vorrichtung nach den Ansprüchen 1, 2, 3 und 4, **dadurch gekennzeichnet, dass** die Magnetquelle aus einem Elektromagneten (1) mit einem Kern (1a) besteht.

6. Vorrichtung nach Anspruch 5, **dadurch gekennzeichnet, dass** zwei parallele gebogene Arme (2) mit Kontaktflächen (2a), die ein Magnetfeld an einer Fläche eines Herzens (15) erzeugen, aus den Enden des Kerns (1a) des Elektromagneten (1) heraus kommen.

7. Vorrichtung nach den Ansprüchen 1, 2, 3, 4, 5 und 6, **dadurch gekennzeichnet, dass** die Arme (2) des das Magnetfeld erzeugenden Teils biegsam sind, wobei eine Basis zum Anlegen des Magnetfelds außerhalb eines Operationsbereiches (12) angeordnet ist, wodurch es ermöglicht wird, die Arme (2) bezüglich ihrer Position relativ zu dem Operationsbereich (12) auszurichten.

8. Vorrichtung nach den Ansprüchen 1, 2, 3, 4, 5, 6 und 7, **dadurch gekennzeichnet, dass** sie aus einem starren kreisförmigen Rahmen (13) aus ferromagnetischem Material besteht, der mit entfernbaren dehnbaren Haken (14) aus magnetisiertem ferromagnetischen Material versehen ist, so dass die Haken (14) und Teile der das Magnetfeld erzeugenden Elemente durch ein induziertes Magnetfeld zu jedem beliebigen Punkt auf dem ferromagnetischen Rahmen (13) gezogen werden.

9. Vorrichtung nach den Ansprüchen 1, 2, 3, 4, 5, 6 und 7, **dadurch gekennzeichnet, dass** die in den Herzmuskel (3) eingebrachten Teile (4a, 4b) aus einer ferromagnetischen Doppelfaser in Form einer Sicherheitsnadel oder ferromagnetischen Halteklammern bestehen können.

## Revendications

1. Dispositif pour la stabilisation locale d'un muscle cardiaque, **caractérisé par le fait qu'**il est constitué par deux parties qui sont indépendantes en ce qui concerne la physique mais dépendantes en ci que concerne la fonction, une première partie comprenant une source magnétique (1, 11) avec des bras courbés (2), les sections finales desquelles forment des surfaces de contact (2a) qui sont montées sur la face extérieure d'un coeur (15) par une distance nécessaire pour l'immobilisation, et une deuxième partie est constituée des fibres (4a, 4b) fabriquées d'une matière ferromagnétique, chaque fibre (4a, 4b) étant introduite par l'intermédiaire d'une aiguille (5) dans le muscle cardiaque (3) par une distance en corrélation avec les surfaces de contact (2a) des bras (2), en sorte que chaque fibre (4a, 4b) est tirée en direction des surfaces de contact (2, 2a) lorsque les surfaces de contact (2a) des bras (2) sont placées au-dessus des fibres (4a, 4b) ferromagnétiques introduites, par quel moyen une immobilisation d'une partie du coeur (15) est obtenue par l'introduction de pour le moins deux fibres (4a, 4b) ferromagnétiques dans la partie du coeur (15) qui doit être immobilisée, chaque fibre (4a, 4b) étant tirée dans une manière indépendante en direction d'un bras (2).

2. Dispositif selon la revendication 1, **caractérisé par le fait que** la source magnétique est constituée d'un aimant permanent (11) qui comprend deux bras (2) formés comme une fourche, non seulement la forme et l'espacement mutuel de ces bras mais aussi la longueur de leur surfaces de contact (2a) déterminent la largeur et la longueur de la partie (17) du coeur (15) qui est immobilisée, chaque fibre (4a, 4b) ferromagnétique est placée avec une distance aux bras (2) bien définie, les deux fibres étant tirées simultanément en direction vers les bras par le champ magnétique appliqué d'aimant permanent (11), grâce à quoi la partie (17) est immobilisée.

3. Dispositif selon les revendications 1 et 2, **caractérisé par le fait que** les surfaces de contact (2a) des bras (2) ont une forme d'un rectangle (8) avec des pièces de raccord (9) non-magnétiques.

4. Dispositif selon la revendication 1 ou 2, **caractérisé par le fait que** les surfaces de contact (2a) des bras (2) ont une forme circulaire (10) avec des pièces de raccord (9) non-magnétiques.

5. Dispositif selon les revendications 1, 2, 3 et 4, **caractérisé par le fait que** la source magnétique est constituée par un électro-aimant (1) avec un tore (1a).

6. Dispositif selon la revendication 5, **caractérisé par le fait que** deux parallèles bras (2) courbés avec des surfaces de contact (2a) qui produisent un champ magnétique à une face d'un coeur (15) sortent des bouts du tore (1a) d'électro-aimant (1).

7. Dispositif selon les revendications 1, 2, 3, 4, 5 et 6, **caractérisé par le fait que** les bras (2) de la partie qui produit le champ magnétique sont flexible, une base pour appliquer le champ magnétique étant arrangée au dehors d'un secteur d'opération (12) grâce à quoi il est possible d'orienter les bras (2) concernant leur position par rapport au secteur d'opération (12).

8. Dispositif selon les revendications 1, 2, 3, 4, 5, 6 et 7, **caractérisé par le fait qu'**il est constitué par un cadre rigide circulaire (13) en matière ferromagnétique qui est pourvu des griffes élastiques (14) en matière ferromagnétique, en sorte que les griffes (14) et quelques parts d'éléments qui produisent le champ magnétique sont tirées à chaque point à volonté sur le cadre (13) ferromagnétique au moyen d'un champ magnétique induit.

9. Dispositif selon les revendications 1, 2, 3, 4, 5, 6 et 7, **caractérisé par le fait que** les parties (4a, 4b) introduites dans le muscle cardiaque (3) peuvent être composées d'une fibre double ferromagnétique en forme d'une épingle de sûreté ou d'un étrier de retenue ferromagnétique.
